# EUROPÄISCHE PATENTSCHRIFT

(11) **EP 4 567 021 B1**
(45) Veröffentlichungstag und Bekanntmachung des Hinweises auf die Patenterteilung: **04.03.2026**
(21) Anmeldenummer: 23215246.2
(22) Anmeldetag: 08.12.2023
(51) Int. Cl.: C07C 67/00, C07C 69/24, C07C 69/608, C07C 69/612, C07C 69/65, C07C 69/74, C07C 69/75, C07C 69/734, C07C 69/753

(54) **VERFAHREN ZUR ALKOXYCARBONYLIERUNG VON ETHYLENISCH UNGESÄTTIGTEN VERBINDUNGEN UNTER EINSATZ VON CO2 UND EINER ZINKVERBINDUNG**
METHOD FOR THE ALKOXYCARBONYLATION OF ETHYLENICALLY UNSATURATED COMPOUNDS USING CO2 AND A ZINC COMPOUND
PROCÉDÉ D'ALCOXYCARBONYLATION DE COMPOSÉS ÉTHYLÉNIQUEMENT INSATURÉS EN UTILISANT DU CO2 ET UN COMPOSÉ DE ZINC

(43) Veröffentlichungstag der Anmeldung: 11.06.2025
(73) Patentinhaber: Evonik Oxeno GmbH & Co. KG, 45772 Marl (DE)
(72) Erfinder: HUANG, Weiheng, 76706 Waco, TX (US); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- CN-A- 115 403 465
- LIPENG WU ET AL: "Ruthenium-catalysed alkoxycarbonylation of alkenes with carbon dioxide", NATURE COMMUNICATIONS, vol. 5, 11 February 2014 (2014-02-11), pages 1 - 6, XP055152708, DOI: 10.1038/ncomms4091

## Beschreibung

Die Erfindung betrifft ein Verfahren zur Alkoxycarbonylierung von ethylenisch ungesättigten Verbindungen unter Einsatz von CO₂ und einer Zinkverbindung.

In EP 4 001 256 A1 wird ein Verfahren zur Alkoxycarbonylierung von ethylenisch ungesättigten Verbindungen unter Einsatz von benzolbasierten Diphosphinliganden und Aluminiumtriflat beschrieben.

In CN 115 403 465 A wird ein Verfahren zur Hestellung von Carboxylaten durch Umsetzung von Olefinen mit CO₂ beschrieben.

Ein Artikel von Wu, L., Liu, Q., Fleischer, I. et al. in Nat Commun 5, 3091 (2014), https://doi.org/10.1038/ ncomms4091, beschreibt eine Ruthenium-katalysierte Alkoxycarbonylierung von Alkenen mit Kohlenstoffdioxid.

Die technische Aufgabe der Erfindung ist die Bereitstellung eines neuen Verfahrens, welches eine gesteigerte Ausbeute an Ester liefern soll.

Die Aufgabe wird gelöst durch ein Verfahren nach Anspruch 1.

Verfahren umfassend die Verfahrensschritte:
a) Vorlegen einer ethylenisch ungesättigten Verbindung;
b) Zugabe eines Liganden gemäß Formel (1): 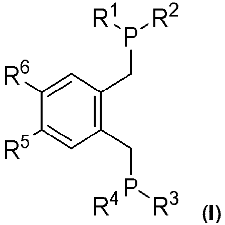 wobei
   R¹, R², R³, R⁴ jeweils für -(C₁-C₁₂)-Alkyl stehen,
   R⁵, R⁶ jeweils für einen Rest stehen ausgewählt aus: -H, -O-(C₁-C₁₂)-Alkyl, -(C₁-C₁₂)-Alkyl,
   und einer Verbindung, welche Pd umfasst;
c) Zugabe einer Zinkverbindung;
d) Zugabe eines Alkohols;
e) Zuführen von CO₂ und H₂;
f) Erwärmen des Reaktionsgemisches aus a) bis e), wobei die ethylenisch ungesättigte Verbindung zu einem Ester umgesetzt wird.

Der Begriff (C₁-C₁₂)-Alkyl umfasst geradkettige und verzweigte Alkylgruppen mit 1 bis 12 Kohlenstoffatomen.

In einer Variante des Verfahrens stehen R¹, R², R³, R⁴ für -(C₁-C₄)-Alkyl.

In einer Variante des Verfahrens stehen R¹, R², R³, R⁴ für *-^{tert}*Bu.

In einer Variante des Verfahrens stehen R⁵, R⁶ für einen Rest, der ausgewählt ist aus: -H, -O-(C₁-C₄)-Alkyl.

In einer Variante des Verfahrens stehen R⁵, R⁶ für einen Rest, der ausgewählt ist aus: -H, -OCH₃.

In einer Variante des Verfahrens weist der Ligand die Struktur (**1**) oder (**2**) auf:

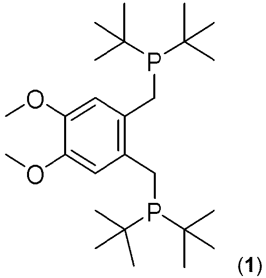

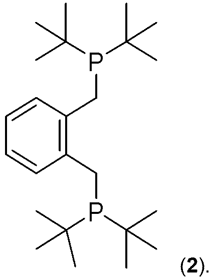

In einer Variante des Verfahrens weist der Ligand die Struktur (**1**) auf:

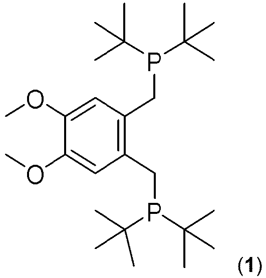

In einer Variante des Verfahrens weist der Ligand die Struktur (**2**) auf:

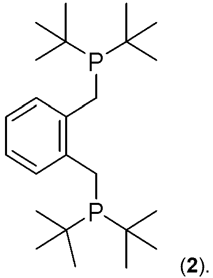

In einer Variante des Verfahrens ist die Verbindung in Verfahrensschritt b), welche Pd umfasst, ausgewählt aus: Palladiumdichlorid, Palladium(II)-acetylacetonat, Palladium(II)-acetat (Pd(OAc)₂), Dichloro(1,5-cyclooctadiene)palladium(II), Bis(dibenzylideneaceton)palladium (Pd(dba)₂), Bis(acetonitrile)dichloropalladium(II), Palladium(cinnamyl)dichlorid

In einer Variante des Verfahrens ist die Verbindung in Verfahrensschritt b), welche Pd umfasst, ausgewählt aus: Pd(dba)₂, Pd(OAc)₂.

In einer Variante des Verfahrens ist die Zinkverbindung ausgewählt aus: Zn(OTf)₂, Zn(SO₃C₆H₄CH₃)·xH₂O, Zn(SO₃CH₃)·xH₂O.

In einer Variante des Verfahrens ist die Zinkverbindung Zn(OTf)₂.

In einer Variante des Verfahrens ist der Alkohol in Verfahrensschritt d) ausgewählt aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol in Verfahrensschritt d) ausgewählt aus: Methanol, Ethanol, 1-Butanol.

In einer Variante des Verfahrens erfolgt das Zuführen von CO₂ mit einem Druck im Bereich von 1 MPa (10 bar) bis 5 MPa (50 bar).

In einer Variante des Verfahrens erfolgt das Zuführen von CO₂ mit einem Druck im Bereich von 1 MPa (10 bar) bis 3 MPa (30 bar).

In einer Variante des Verfahrens erfolgt das Zuführen von H₂ mit einem Druck im Bereich von 1 MPa (10 bar) bis 5 MPa (50 bar).

In einer Variante des Verfahrens erfolgt das Zuführen von H₂ mit einem Druck im Bereich von 1 MPa (10 bar) bis 3 MPa (30 bar).

In einer Variante des Verfahrens erfolgt das Erwärmen auf eine Temperatur im Bereich von 80 °C bis 160 °C.

In einer Variante des Verfahrens erfolgt das Erwärmen auf eine Temperatur im Bereich von 100 °C bis 140 °C.

Im Folgenden soll die Erfindung anhand von Ausführungsbeispielen näher erläutert werden.

### Variation des Additives

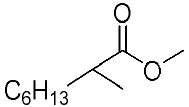

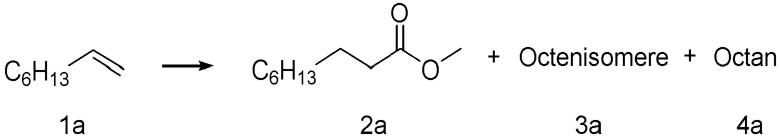

Die festen Ausgangsstoffe Palladiumvorstufe Pd(dba)₂ (2,9 mg, 0,005 mmol), Ligand (**2**) 1,2-Bis(di-tert-butylphosphinomethyl)benzol (d^{t}bpx) (0,01 mmol) und Additiv X (0,05 mmol) wurden abgewogen und im Handschuhkasten in ein 12-ml-Schraubdeckel-Vial mit 6-mm-Magnetrührstab gegeben. Das Vial wurde mit einem Teflon/Silikon-Septum und einem Phenolharzdeckel verschlossen und dann aus dem Handschuhkasten ausgetragen. Das Vial wurde mit einer Nadel unter Argonstrom an eine Schlenklinie angeschlossen. Mit einer normalen Spritze bzw. einer Mikroliterspritze wurden destilliertes Methanol (2,0 ml) und destilliertes 1-Octen (157 µl, 1,0 mmol) eingespritzt. Das Vial wurde auf einer Legierungsplatte gehalten und in einen Autoklaven (300 ml) der Reihe 4560 von Parr gegeben. Der Autoklav wurde bei Raumtemperatur zweimal mit Stickstoffgas gespült. Außerdem wurde zweimal mit Kohlendioxid gespült und Kohlendioxid bis 20 bar aufgepresst, wonach Wasserstoff (mit 20 bar) bis zu einem Gesamtdruck von 40 bar zugeführt wurde. Der Ansatz wurde auf 140 °C erhitzt und 20 Stunden gerührt (600 U/min). Danach wurde der Autoklav mit Eiswasser abgekühlt, wonach das Gas vorsichtig abgelassen wurde. Die Lösung wurde mit Isooctan (83 µl, 0,5 mmol) als internem Standard versetzt, über Celite filtriert und durch Gaschromatographie analysiert.

### Reaktionsbedingungen:

Pd(dpa)₂ (0,005 mmol), Ligand **(2)** d^{t}bpx (0,01 mmol), Additiv (variable) (0,05 mmol), MeOH, CO₂ (20 bar), H₂ (20 bar), 140 °C, 20 h

**Tabelle 1:**

| Additiv | Ausbeute 2a | n:iso | Ausbeute 3a + 4a |
|---|---|---|---|
| Zn(OTf)₂ | 25 % | 92 : 8 | 74 % |
| Zn(SO₃C₆H₄CH₃)·xH₂O | 14 % | 92 : 8 | 85 % |
| Zn(SO₃CH₃)·xH₂O | 23 % | 94 : 6 | 76 % |
| PTSA·H₂O | - | - | 98 % |
| Al(OTf)₃ | - | - | 98 % |
| Fe(OTf)₃ | - | - | 98 % |

Bei den Versuchen mit PTSA·H₂O, Al(OTf)₃ und Fe(OTf)₃ handelt es sich um Vergleichsversuche.

### Variation des Liganden

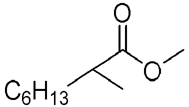

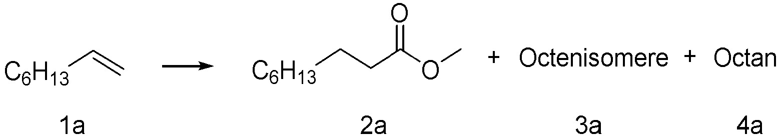

Die Versuchsreihe wurde analog zu der *Variation des Additives* durchgeführt, nur dass als Additiv in dieser Reihe immer Zn(OTf)₂ verwendet wurde, und der Ligand variiert. Von dem Liganden wurden immer 0,01 mmol eingesetzt.

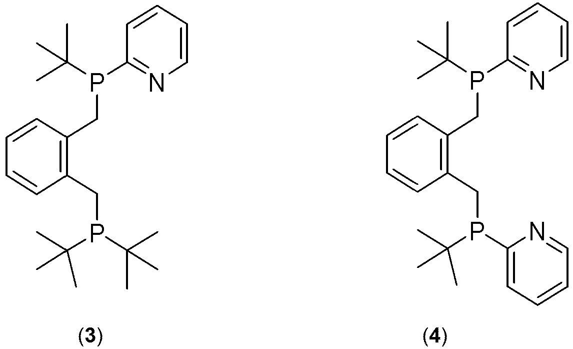

### Reaktionsbedingungen:

Pd(dpa)₂ (0,005 mmol), Ligand (variable) (0,01 mmol), Additiv: Zn(OTf)₂ (0,05 mmol), MeOH, CO₂ (20 bar), H₂ (20 bar), 140 °C, 20 h

**Tabelle 2:**

| Ligand | Ausbeute 2a | n:iso | Ausbeute 3a + 4a |
|---|---|---|---|
| **(2)** | 25 % | 92 : 8 | 74 % |
| **(1)** | 33 % | 92 : 8 | 65 % |
| **(3)** | 2 % | - | 97 % |
| **(4)** | < 1 % | - | 98 % |

Bei den Versuchen mit Ligand **(3)** und **(4)** handelt es sich um Vergleichsversuche.

### Variation der ethylenisch ungesättigten Verbindung / des Alkohols

Die Versuchsreihe wurde entsprechend zu den zuvor beschriebenen Versuchen durchgeführt mit den folgenden Reaktionsbedingungen:

### Reaktionsbedingungen:

Pd(OAc)₂ (0,03 mmol), Ligand **(2)** d^{t}bpx (0,06 mmol), Additiv: Zn(OTf)₂ (0,05 mmol), MeOH, CO₂ (20 bar), H₂ (20 bar), 120 °C, 45 h

**Tabelle 3: Methoxycarbonylierung**

| ungesättigten Verbindung(en) / Produkt(e) | Ausbeute | n:iso |
|---|---|---|
| 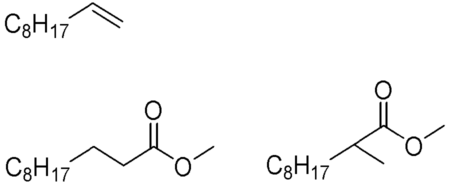 | 83 % | 93 : 7 |
| 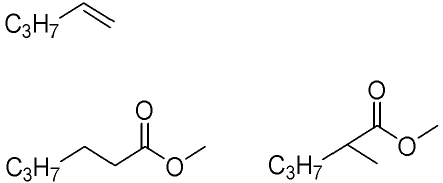 | 69 % | 93 : 7 |
| 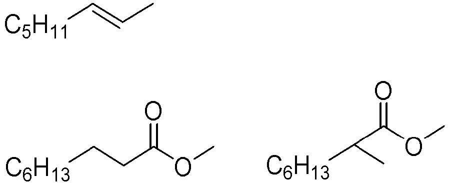 | 82 % | 93 : 7 |
| 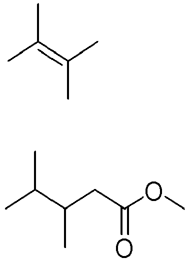 | 12 % | |
| 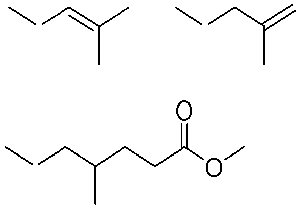 | 47 % | |
| 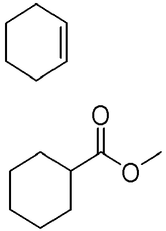 | 44 % | |
| 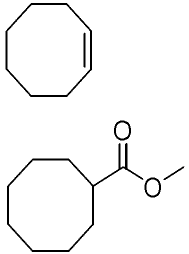 | 25 % | |
| 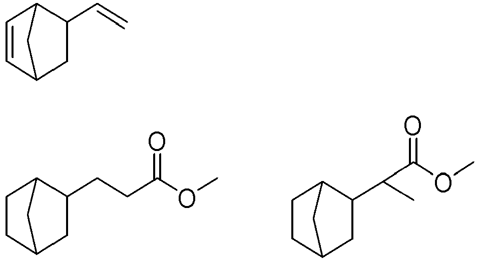 | 42 % | 62 : 38 |
| 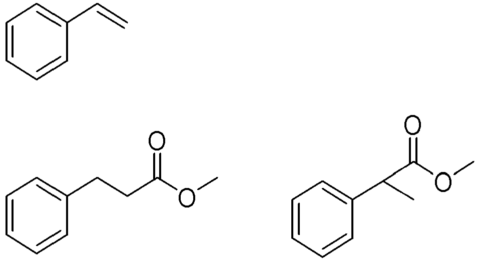 | 15 % | 99 : 1 |
| 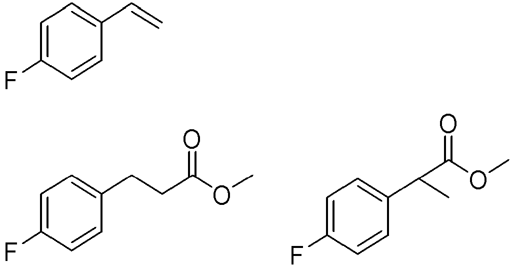 | 18 % | 99 : 1 |
| 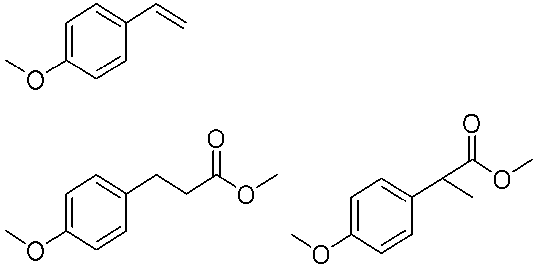 | 17 % | 99 : 1 |
| 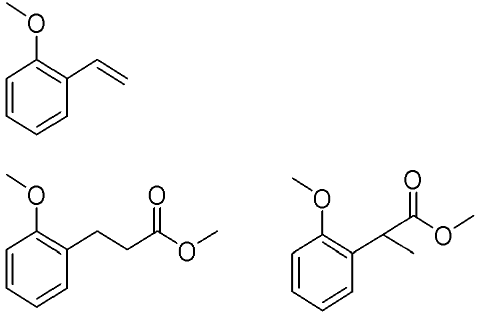 | 8 % | 99 : 1 |
| 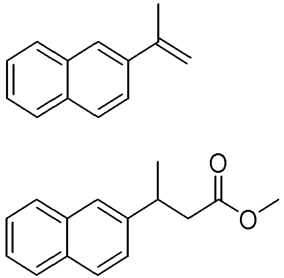 | 21 % | |
| 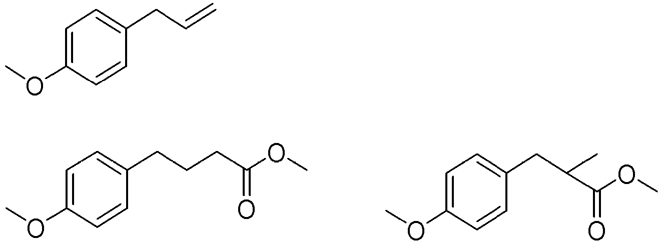 | 32 % | 98 : 2 |
| 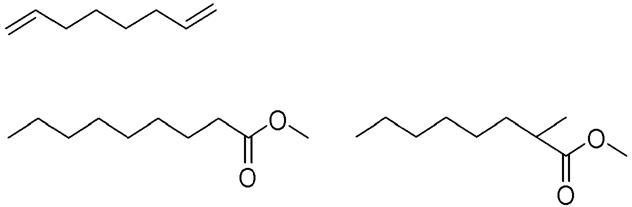 | 14 % | 90 : 10 |
| 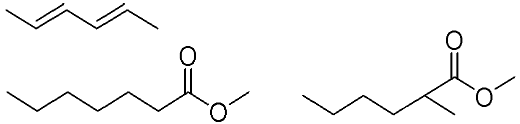 | 21 % | 80 : 20 |

In der nachfolgenden Versuchsreihe wurde Ethanol an Stelle von Methanol eingesetzt.

### Reaktionsbedingungen:

Pd(OAc)₂ (0,03 mmol), Ligand **(2)** d^{t}bpx (0,06 mmol), Additiv: Zn(OTf)₂ (0,05 mmol), EtOH, CO₂ (20 bar), H₂ (20 bar), 120 °C, 45 h

**Tabelle 4: Ethoxycarbonylierung**

| ungesättigten Verbindung(en) / Produkt(e) | Ausbeute | n:iso |
|---|---|---|
| 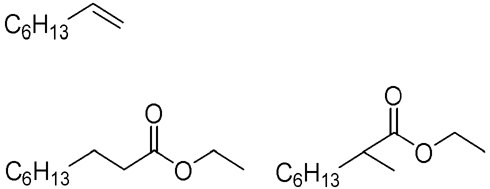 | 68 % | 93 : 7 |
| 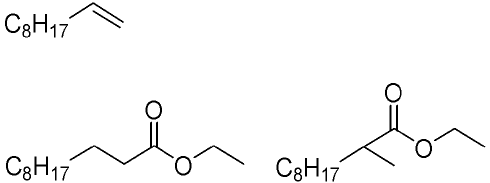 | 66 % | 94 : 6 |
| 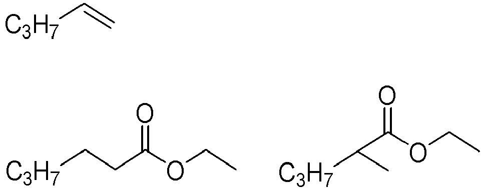 | 54 % | 94 : 6 |
| 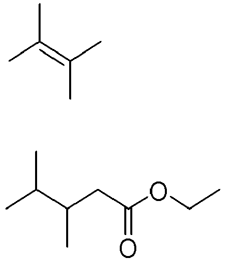 | 16 % | |
| 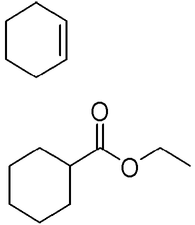 | 30 % | |
| 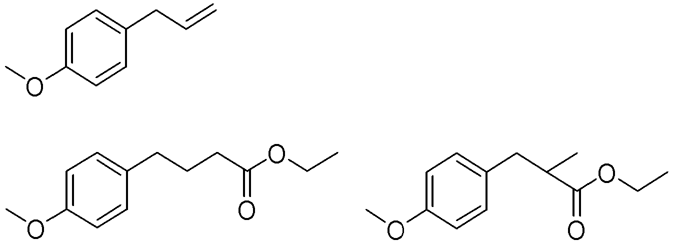 | 29 % | 98 : 2 |

In der nachfolgenden Versuchsreihe wurde als Alkohol 1-Butanol eingesetzt.

### Reaktionsbedingungen:

Pd(OAc)₂ (0,03 mmol), Ligand **(2)** d^{t}bpx (0,06 mmol), Additiv: Zn(OTf)₂ (0,05 mmol), 1-Butanol, CO₂ (20 bar), H₂ (20 bar), 120 °C, 45 h

**Tabelle 5: Butyloxycarbonylierung**

| ungesättigten Verbindung(en) / Produkt(e) | Ausbeute | n:iso |
|---|---|---|
| 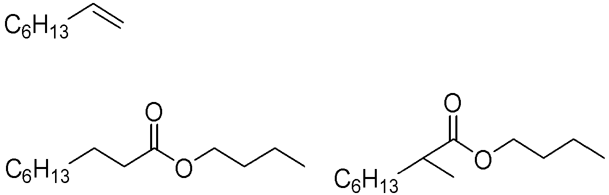 | 28 % | 92 : 8 |
| 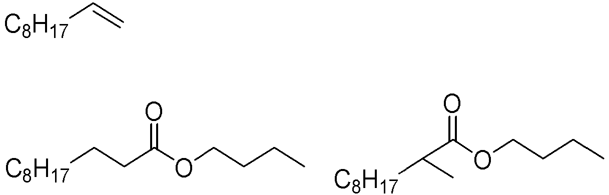 | 21 % | 92 : 8 |
| 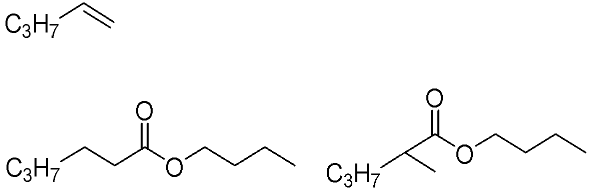 | 23 % | 92 : 8 |

Wie die durchgeführten Versuche zeigen, wird die gestellte Aufgabe durch ein erfindungsgemäßes Verfahren gelöst.

## Patentansprüche

1. Verfahren umfassend die Verfahrensschritte:
a) Vorlegen einer ethylenisch ungesättigten Verbindung;
b) Zugabe eines Liganden gemäß Formel (**I**): 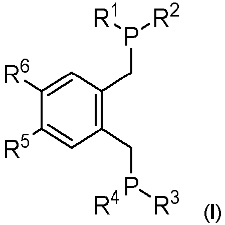 wobei
R¹, R², R³, R⁴ jeweils für -(C₁-C₁₂)-Alkyl stehen,
R⁵, R⁶ jeweils für einen Rest stehen ausgewählt aus: -H, -O-(C₁-C₁₂)-Alkyl, -(C₁-C₁₂)-Alkyl,
und einer Verbindung, welche Pd umfasst;
c) Zugabe einer Zinkverbindung;
d) Zugabe eines Alkohols;
e) Zuführen von CO₂ und H₂;
f) Erwärmen des Reaktionsgemisches aus a) bis e), wobei die ethylenisch ungesättigte Verbindung zu einem Ester umgesetzt wird.

2. Verfahren nach Anspruch 1,
wobei R¹, R², R³, R⁴ für -(C₁-C₄)-Alkyl stehen.

3. Verfahren nach einem der Ansprüche 1 bis 2,
wobei R⁵, R⁶ für einen Rest stehen ausgewählt aus: -H, -O-(C₁-C₄)-Alkyl.

4. Verfahren nach einem der Ansprüche 1 bis 3,
wobei der Ligand die Struktur **(1)** oder **(2)** aufweist:

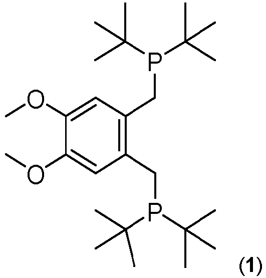

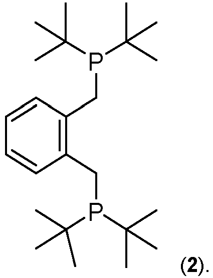

5. Verfahren nach einem der Ansprüche 1 bis 4,
wobei der Ligand die Struktur **(1)** aufweist:

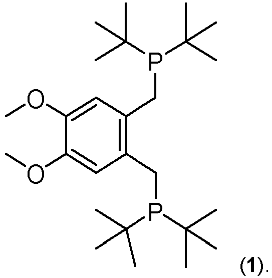

6. Verfahren nach einem der Ansprüche 1 bis 4,
wobei der Ligand die Struktur **(2)** aufweist:

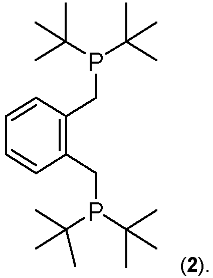

7. Verfahren nach einem der Ansprüche 1 bis 6,
wobei die Verbindung in Verfahrensschritt b), welche Pd umfasst, ausgewählt ist aus:
Palladiumdichlorid, Palladium(II)-acetylacetonat, Palladium(II)-acetat (Pd(OAc)₂), Dichloro(1,5-cyclooctadiene)palladium(II), Bis(dibenzylideneaceton)palladium (Pd(dba)₂), Bis(acetonitrile)dichloropalladium(II), Palladium(cinnamyl)dichlorid

8. Verfahren nach einem der Ansprüche 1 bis 7,
wobei die Verbindung in Verfahrensschritt b), welche Pd umfasst, ausgewählt ist aus: Pd(dba)₂, Pd(OAc)₂.

9. Verfahren nach einem der Ansprüche 1 bis 8,
wobei die Zinkverbindung ausgewählt ist aus: Zn(OTf)₂, Zn(SO₃C₆H₄CH₃)·xH₂O, Zn(SO₃CH₃)·xH₂O.

10. Verfahren nach einem der Ansprüche 1 bis 9,
wobei die Zinkverbindung Zn(OTf)₂ ist.

11. Verfahren nach einem der Ansprüche 1 bis 10,
wobei der Alkohol in Verfahrensschritt d) ausgewählt ist aus: Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

12. Verfahren nach einem der Ansprüche 1 bis 11,
wobei der Alkohol in Verfahrensschritt d) ausgewählt ist aus: Methanol, Ethanol, 1-Butanol.

13. Verfahren nach einem der Ansprüche 1 bis 12,
wobei das Zuführen von CO₂ mit einem Druck erfolgt im Bereich von 1 MPa (10 bar) bis 5 MPa (50 bar).

14. Verfahren nach einem der Ansprüche 1 bis 13,
wobei das Zuführen von H₂ mit einem Druck erfolgt im Bereich von 1 MPa (10 bar) bis 5 MPa (50 bar).

15. Verfahren nach einem der Ansprüche 1 bis 14,
wobei das Erwärmen auf eine Temperatur erfolgt im Bereich von 80 °C bis 160 °C.

## Claims

1. Process comprising the process steps of:
a) initially charging an ethylenically unsaturated compound;
b) adding a ligand of formula (I): 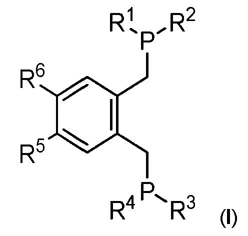 where
R¹, R², R³, R⁴ are each -(C₁-C₁₂)-alkyl,
R⁵, R⁶ are each a radical selected from: -H, -O-(C₁-C₁₂)-alkyl, -(C₁-C₁₂)-alkyl,
and a compound comprising Pd;
c) adding a zinc compound;
d) adding an alcohol;
e) feeding in CO₂ and H₂;
f) heating the reaction mixture of a) to e), with conversion of the ethylenically unsaturated compound to an ester.

2. Process according to Claim 1,
where R¹, R², R³, R⁴ are -(C₁-C₄)-alkyl.

3. Process according to either of Claims 1 and 2,
where R⁵, R⁶ are a radical selected from: -H, -O-(C₁-C₄)-alkyl.

4. Process according to any of Claims 1 to 3,
wherein the ligand has the structure (1) or (2):

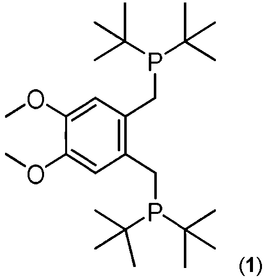

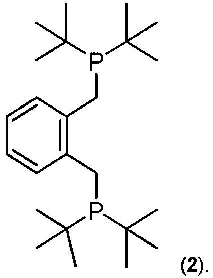

5. Process according to any of Claims 1 to 4,
wherein the ligand has the structure (1):

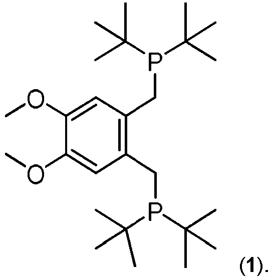

6. Process according to any of Claims 1 to 4,
wherein the ligand has the structure (2):

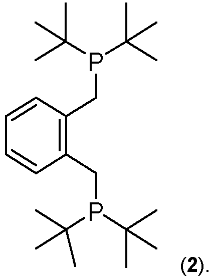

7. Process according to any of Claims 1 to 6,
wherein the compound in process step b) which comprises Pd is selected from: palladium dichloride, palladium(II) acetylacetonate, palladium(II) acetate (Pd(OAc)₂), dichloro(1,5-cyclooctadiene)palladium(II), bis(dibenzylideneacetone)palladium (Pd(dba)₂), bis(acetonitrile)dichloropalladium(II), palladium(cinnamyl) dichloride.

8. Process according to any of Claims 1 to 7,
wherein the compound in process step b) which comprises Pd is selected from: Pd(dba)₂, Pd(OAc)₂.

9. Process according to any of Claims 1 to 8,
wherein the zinc compound is selected from: Zn(OTf)₂, Zn(SO₃C₆H₄CH₃)·xH₂O, Zn(SO₃CH₃)·xH₂O.

10. Process according to any of Claims 1 to 9,
wherein the zinc compound is Zn(OTf)₂.

11. Process according to any of Claims 1 to 10,
wherein the alcohol in process step d) is selected from: methanol, ethanol, 1-propanol, 1-butanol, 1-pentanol, 1-hexanol, 2-propanol, tert-butanol, 3-pentanol, cyclohexanol, phenol, or mixtures thereof.

12. Process according to any of Claims 1 to 11,
wherein the alcohol in process step d) is selected from: methanol, ethanol, 1-butanol.

13. Process according to any of Claims 1 to 12,
wherein CO₂ is fed in with a pressure in the range from 1 MPa (10 bar) to 5 MPa (50 bar).

14. Process according to any of Claims 1 to 13,
wherein H₂ is fed in with a pressure in the range from 1 MPa (10 bar) to 5 MPa (50 bar).

15. Process according to any of Claims 1 to 14,
wherein the reaction mixture is heated to a temperature in the range from 80°C to 160°C.

## Revendications

1. Procédé comprenant les étapes de procédé :
a) présentation d'un composé éthyléniquement insaturé ;
b) ajout d'un ligand selon la formule (I) : 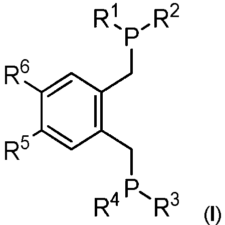
R¹, R², R³, R⁴ représentant chacun -alkyle (C₁-C₁₂),
R⁵, R⁶ représentant chacun un radical choisi parmi : -H, -O-alkyle(C₁-C₁₂), -alkyle (C₁-C₁₂),
et un composé, qui comprend du Pd ;
c) ajout d'un composé de zinc ;
d) ajout d'un alcool ;
e) introduction de CO₂ et H₂ ;
f) chauffage du mélange réactionnel issu de a) à e), le composé éthyléniquement insaturé étant converti en un ester.

2. Procédé selon la revendication 1,
R¹, R², R³, R⁴ représentant -alkyle(C₁-C₄).

3. Procédé selon l'une des revendications 1 à 2,
R⁵, R⁶ représentant un radical choisi parmi : -H, -O-alkyle(C₁-C₄).

4. Procédé selon l'une des revendications 1 à 3,
le ligand comportant la structure (1) ou (2) :

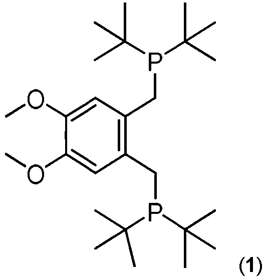

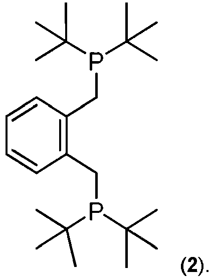

5. Procédé selon l'une des revendications 1 à 4,
le ligand comportant la structure (1) :

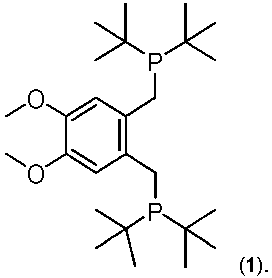

6. Procédé selon l'une des revendications 1 à 4,
le ligand comportant la structure (2) :

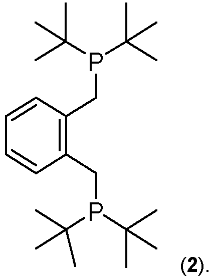

7. Procédé selon l'une des revendications 1 à 6,
le composé dans l'étape de procédé b), qui comprend du Pd, étant choisi parmi : dichlorure de palladium, acétylacétonate de palladium(II), acétate de palladium(II) (Pd(OAc)₂), dichloro(1,5-cyclooctadiène)palladium(II), bis(dibenzylidèneacétone)palladium (Pd(dba)₂), bis(acétonitrile)dichloropalladium(II), dichlorure de palladium(cinnamyl)palladium.

8. Procédé selon l'une des revendications 1 à 7,
le composé dans l'étape de procédé b), qui comprend du Pd, étant choisi parmi : Pd(dba)₂, Pd(OAc)₂.

9. Procédé selon l'une des revendications 1 à 8,
le composé de zinc étant choisi parmi : Zn(OTf)₂, Zn(SO₃C₆H₄CH₃)·xH₂O, Zn(SO₃CH₃)·xH₂O.

10. Procédé selon l'une des revendications 1 à 9,
le composé de zinc étant Zn(OTf)₂.

11. Procédé selon l'une des revendications 1 à 10,
l'alcool dans l'étape de procédé d) étant choisi parmi : méthanol, éthanol, 1-propanol, 1-butanol, 1-pentanol, 1-hexanol, 2-propanol, tert-butanol, 3-pentanol, cyclohexanol, phénol ou leurs mélanges.

12. Procédé selon l'une des revendications 1 à 11,
l'alcool dans l'étape de procédé d) étant choisi parmi : méthanol, éthanol, 1-butanol.

13. Procédé selon l'une des revendications 1 à 12,
l'introduction de CO₂ étant effectuée à une pression dans la plage de 1 MPa (10 bar) à 5 MPa (50 bar).

14. Procédé selon l'une des revendications 1 à 13,
l'introduction de H₂ étant effectuée à une pression dans la plage de 1 MPa (10 bar) à 5 MPa (50 bar).

15. Procédé selon l'une des revendications 1 à 14,
le chauffage étant effectué à une température dans la plage de 80 °C à 160 °C.
